# EUROPEAN PATENT SPECIFICATION

(11) **EP 3 415 500 B1**
(45) Date of publication and mention of the grant of the patent: **01.04.2020**
(21) Application number: 18176995.1
(22) Date of filing: 11.06.2018
(51) Int. Cl.: C07D 207/267

(54) **METHOD FOR PREPARING METHYLPYRROLIDONES**
VERFAHREN ZUR HERSTELLUNG VON METHYLPYRROLIDONES
PROCÉDÉ POUR LA PRÉPARATION DE MÉTHYLPYRROLIDONES

(30) Priority: 12.06.2017 GB 201709297
(43) Date of publication of application: 19.12.2018
(73) Proprietor: Rheinisch-Westfälische Technische Hochschule (RWTH) Aachen, 52056 Aachen (DE)
(72) Inventor: PALKOVITS, Regina, 52074 Aachen (DE); HAUSOUL, Peter, 6371 GE Landgraaf (NL); SCHUTE, Kai, 52064 Aachen (DE)
(74) Representative: Michalski Hüttermann & Partner Patentanwälte mbB

(56) References cited:
- WO-A1-2010/063617
- GB-A- 1 494 454

## Description

The present invention relates to a method for preparing 3-methylpyrrolidin-2-one and 4-methylpyrrolidin-2-one by reductive amination of itaconic acid.

Biogenic platform chemicals, which are made from renewable raw materials, are of considerable economic importance and are intensively sought-after to replace conventionally produced platform chemicals such as pyrrolidones.

The general process of reductive amination to yield pyrrolidones is known in the prior art. The reductive amination of succinic acid to yield unsubstituted pyrrolidone was disclosed in DE 1204674. Itaconic acid was disclosed as a starting material using primary amines for the preparation of methyl-substituted N-alkylpyrrolidones in WO 2010/063617. Itaconic acid is a biogenic platform chemical on the basis of renewable raw material such as cellulosic feedstocks. In view of a terminal double bond in the side chain and its reactivity, and also the possibility of toxic side products, itaconic acid however is rarely considered as a starting material. GB 1494454 discloses a process for preparing a 2-pyrrolidone by hydrogenating succinonitrile or a substituted succinonitrile in the liquid phase in the presence of ammonia at a hydrogen partial pressure of between 1 to 50 atmospheres, and hydrolysing the resulting reaction product with water.

Due to the importance of pyrrolidones there is a constant need for improvements and alternative preparation methods of pyrrolidones from biogenic acids. It was thus an object of the present invention to provide a method for preparing methylpyrrolidones from itaconic acid with high yield.

The object is achieved in the present invention by a method for preparing methylpyrrolidones, the method comprising the steps of:
a) providing itaconic acid or a derivative of itaconic acid selected from the group of its salts, esters, imids, amids and anhydrids, and
b) reductive amination of itaconic acid or its derivative in the presence of hydrogen and a hydrogenation catalyst,
   wherein itaconic acid or its derivative is reacted with NH₃ to yield 3-methylpyrrolidin-2-one and/or 4-methylpyrrolidin-2-one, and
   wherein the hydrogenation catalyst is a metal catalyst selected from the group consisting of nickel, palladium, platinum, rhodium, ruthenium and combinations thereof.

It has been found that reacting itaconic acid with gaseous NH₃ yields pyrrolidin-2-ones with a methyl substituent in positions 3 or 4 resulting from the substrate with surprisingly high yields. The reaction with gaseous NH₃ further was found to provide a clean and efficient reduction method. Gaseous NH₃ provides a high mobility in the gas phase and also in solution, which can provide a fast reaction. The use of gaseous NH₃ particularly provides a clean processing as of the low energy demanding subsequent down-stream processes. Further, gaseous NH₃ reduces the acidity of the system, which subsequently leads to more robust catalysis steps, as catalyst stability is highly inhibited in acidic medium, particularly in the presence of carboxylic groups. Furthermore, cheaper construction materials for the process design are enabled due to the acid neutralization.

The term "reductive amination" in the sense of the present invention refers to a reaction inducing an amino group into a molecule by a reduction and hydrogenation reaction. The term "catalyst" in the sense of the present invention refers to a substance that affects the rate of a reaction but not the equilibrium, and itself emerges from the reaction chemically unchanged. The term "hydrogenation catalyst" refers to a catalyst enhancing a hydrogenation and/or reduction reaction. The catalyst in the method is a solid compound, while the substrate itaconic acid may be solved or suspended in a solvent or be present solvent-free in its molten state.

The method relates to the synthesis of 3-methylpyrrolidin-2-one and/or 4-methylpyrrolidin-2-one from a reaction of itaconic acid and ammonia in the presence of hydrogen and a hydrogenation catalyst. Itaconic acid may be obtained from renewable raw materials particularly biobased, renewable cellulosic feedstocks. A use of bio-derived itaconic acid as substrate advantageously reduces the cost of manufacture of the resulting methylpyrrolidones relative to conventional methods. Usually, a mixture of 3-methylpyrrolidone and 4-methylpyrrolidone results from the reaction. The product mixture may be separated by methods customarily employed for such separations such as distillation, rectification or crystalization. As used herein, 3-methylpyrrolidone and 3-methylpyrrolidin-2-one, and 4-methylpyrrolidone and 4-methylpyrrolidin-2-one, respectively, are used synonymous.

Also the salts and further derivatives of itaconic acid, such as the anhydrids, esters, amides or imides thereof, may react under the conditions of the reductive amination with ammonia. The term itaconic acid "derivative" as used herein especially refers to the salts, esters, imids, amids and anhydrids of itaconic acid. Itaconic acid comprises two carboxylic groups and may be present in the form of its salts. Salts of itaconic acid may be inorganic or organic salts, for example, metal salts of itaconic acid, such as alkali metal salts or alkaline earth metal salts of itaconic acid. A preferred salt is the ammonium salt of itaconic acid. Itaconic acid further may be used in the form of its anhydride. Further preferred itaconic acid derivatives are itaconic acid esters. Esters may be alkyl esters, for example comprising alkyl groups having from 1 to 6 carbon atoms, preferably comprising alkyl groups having 1 or 2 carbon atoms. Preferred esters of itaconic acid are dimethyl itaconate, diethyl itaconate, 4-methyl itaconate and 4-ethyl itaconate. Further preferred itaconic acid derivatives are itaconamides such as itaconic acid 4-amide and itaconimides.

In view of itaconic acid ammonia can be used in stoichiometric amounts or in excess. A molar ratio of NH₃ to itaconic acid or its derivative in a range from ≥ 1:2 to ≤ 25:1, particularly in a range from ≥ 1:1 to ≤ 2:1, is preferred. A molar ratio of NH₃ to itaconic acid or its derivative of about 1:1 is particularly preferred. A respective amount of NH₃ may be charged on the reactor at the start of the reaction or in a previous reaction step. In small reaction vessels, gaseous NH₃ may for example be pressed only once onto the reaction vessel, while with correspondingly larger reactors, either the pressure may be kept constant or the reaction with NH₃ may be carried out before hydrogen is injected.

The reductive amination of itaconic acid or its derivative takes place in the presence of a hydrogenation catalyst. In embodiments the hydrogenation catalyst is a metal catalyst selected from the group consisting of nickel, palladium, platinum, rhodium, ruthenium and combinations thereof. The catalysts may be used in the form of doped or alloyed metals of several elements, or include a chemical promoter augmenting the activity of a catalyst. The hydrogenation catalyst preferably is selected from the group consisting of palladium, platinum, rhodium and ruthenium. Rhodium and ruthenium are most preferred. Rhodium and ruthenium achieved advantageously high yields in embodiments of the method in solvent and in substrat melt.

The catalyst used in the method may be supported or unsupported. A supported catalyst is one in which the active catalyst agent is deposited on a support material. An unsupported catalyst may be a sponge catalyst, a so-called Raney catalyst such as Raney nickel. The term "Raney catalyst" as used herein refers to catalysts that have a high surface area due to selectively leaching an alloy containing the active metal and a leachable metal such as aluminum. The term Raney catalyst is meant as a generic term as usually used in the field of the invention, not to refer to a particular source. The hydrogenation catalyst preferably is supported to form a supported metal catalyst. The catalyst support material may be selected from the group consisting of carbon, aluminum oxide, titanium oxide, cer-oxide, zirconium oxide, silicon oxide, silica-alumina, zeolites and combinations thereof. A preferred support is selected from carbon or aluminum oxide. Supported hydrogenation catalysts and Raney catalyst are commercially available or may be obtained by established procedures such as impregnation and precipitation methods.

Generaly, the method may be carried out in batch mode or in continuous mode. Suitable reactors are thus both stirred tank reactors and tubular reactors, the reactor used in each case may be selected for the desired reaction conditions, such as temperature, pressure and residence time. In the case of batchwise performance, the reaction may be performed in a high-pressure stirred tank reactor or a reactor cascade. In the case of continuous performance, the reaction may be performed in a fixed bed reactor or slurry reactor with a subsequent catalyst separation.

The reductive amination of itaconic acid or its derivative may be performed in the presence of a solvent or may be performed free of solvent using a substrate melt. It is particularly advantageous for a large-scale production of methylpyrrolidones from biogenic sources that the method can be carried out in an aqueous medium or solvent-free in the substrate melt.

According to a preferred embodiment, the reductive amination of itaconic acid or its derivative is performed in the presence of a solvent. The solvent may be selected from the group consisting of water, alcohols, ethers, dimethylformamide, ammonia, ammonium hydroxide, pyrrolidones, 3-methylpyrrolidin-2-one, 4-methylpyrrolidin-2-one or mixtures thereof. Aprotic solvents may be preferred, such as acetonitrile, furane based solvents or pyrrolidone based solvents and ethers. Also alcohols such as methanol, ethanol, n-propanol, isopropanol, n-butanol and isobutanol may be used. Preferred ethers are selected from tetrahydrofurane (THF) and 2-methyltetrahydrofurane. 2-methyltetrahydrofurane is generally used as a higher boiling substitute for tetrahydrofurane. A further preferred solvent is pyrrolidone.

Further preferred solvents are 3-methylpyrrolidone, 4-methylpyrrolidone and mixtures thereof. Being the reaction products, 3-methylpyrrolidone and 4-methylpyrrolidone advantageously may be circulated in the process. Thus, a continuous reaction system using 3-methylpyrrolidone or 4-methylpyrrolidone is possible. In a continuous reaction system using the product as solvent no recycling or expensive disposal of additional organic solvents may be required.

A further preferred solvent is water. Itaconic acid generally is not easily soluble, but dissolves or suspends in water depending on the concentration. The method thus may be performed in aqueous solution. Solid catalysts can be easily separated after the reaction, for example by simple filtration, sedimentation or centrifugation, and the reaction mixture can be further processed. A simple and technically viable access to the methylpyrrolidones can be provided with good yield and selectivity.

In embodiments of the method performed in a solvent, the hydrogenation catalyst is selected from ruthenium and rhodium. A most preferred hydrogenation catalyst is ruthenium. Ruthenium and rhodium provided best results in view of a high yield of 3- and 4-methylpyrrolidones in aqueous solution. Ruthenium and rhodium may be supported, for example on carbon or aluminum oxide. In embodiments in a solvent, a most preferred hydrogenation catalyst is ruthenium on aluminum oxide or ruthenium on carbon. Ruthenium on carbon is particularly stable in solvents such as in aqueous solution even at high temperatures.

In embodiments of the method performed in a solvent, the hydrogenation reaction may be performed at a temperature in a range from ≥ 150 °C to ≤ 300 °C, preferably in a range from ≥ 180°C to ≤ 200°C. In these temperature ranges high yields could be achieved. It was found that the temperature, in addition to the hydrogenation catalyst and the hydrogen pressure, had considerable influence on the yield of the reaction. Particularly temperatures about 200 °C provided excellent yields of 3- and 4-methylpyrrolidones.

A further parameter investigated was the reaction time. Reaction times in the range from ≥ 2 h to ≤ 32 h, particularly from ≥ 2 h to ≤ 10 h, were found to be advantageous, in particular with respect to the efficiency and economy of the reaction. It was found that a reaction time from 10 to 17 hours resulted in an almost complete conversion of itacomic acid to the methylpyrrolidone products in aqueous solution.

In the method, gaseous ammonia (NH3) is used. NH₃ may be used in technical grade purity, using pure NH₃ or essentially pure NH₃. In embodiments of the method performed in a solvent, NH₃ gas is applied with a pressure in a range from ≥ 0.5 bar to ≤ 50 bar, preferably in a range from ≥ 1 bar to ≤ 10 bar. The given pressure corresponds to the pressed-in pressure at ambient conditions corresponding to 20 ± 2 °C.

Particularly in embodiments of the method performed in a solvent, a molar ratio of NH₃ to itaconic acid or its derivative in a range from ≥ 1:1 to ≤ 2:1, is preferred. A molar ratio of NH₃ to itaconic acid or its derivative of about 1:1 is particularly preferred.

The reductive amination of itaconic acid or its derivative is performed using hydrogen. Hydrogen may be used in technical grade purity, using pure hydrogen or essentially pure hydrogen. In embodiments of the method performed in a solvent, the hydrogen is applied with a pressure in a range from ≥ 50 bar to ≤ 300 bar, preferably in a range from ≥ 150 bar to ≤ 200 bar. The pressure may be maintained or controlled generally via a metered addition of hydrogen.

In preferred embodiments, the reductive amination of itaconic acid or its derivative in a solvent is performed as a one-pot reaction. The term "one-pot reaction" as used herein refers to a reaction or sequence of reactions where the reactant is subjected to successive chemical reactions in just one reactor or vessel. This is advantageous and avoids lengthy separation processes and purifications of intermediates, thus saving time and resources while increasing the yield. The reductive amination of itaconic acid or its derivative in a solvent enables the preparation of 3- and 4-methylpyrrolidones from itaconic acid or derivatives thereof, without need to separate or hydrogenate intermediates in a separate reaction stage. Since the preparation is simple to implement in technical terms, the embodiment provides high process economy. Particularly embodiments of the present invention performed in a solvent provide the advantage that the reaction can be performed without the need of sophisticated equipment. In a further embodiment of the method, the reductive amination of itaconic acid or its derivative may be performed free of solvent and/or using the substrate in molten state. In solvent-free embodiments itaconic acid or its derivative in solid phase may be contacted with gaseous NH₃. The following hydrogenation of the ammonium salt of itaconic acid preferably is performed using the itaconic acid ammonium salt as a substrate melt.

In these embodiments of the method, the method comprises the steps of:
b1) contacting itaconic acid or its derivative in solid phase with gaseous NH₃ to yield the ammonium salt of itaconic acid; and
b2) reacting the ammonium salt of itaconic acid in the molten state with hydrogen in the presence of a hydrogenation catalyst to yield 3-methylpyrrolidin-2-one and/or 4-methylpyrrolidin-2-one.

The step of contacting itaconic acid or its derivative in solid phase with gaseous NH₃ may be performed at a temperature in a range from ≥ 0 °C to ≤ 200 °C, preferably in a range from ≥ 50°C to ≤ 150°C. At these temperatures itaconic acid and the ammonium salt of itaconic acid remain in a solid state.

In these embodiments of the method, the hydrogenation reaction in step b2) may be performed at a temperature in a range from ≥ 150 °C to ≤ 250 °C, preferably in a range from ≥ 150°C to ≤ 200°C. Temperatures in such ranges are above the melting temperature of the ammonium salt of itaconic acid.

It is assumed that the reductive amination of itaconic acid proceeds in two reaction steps, where in a first step the ammonium salt of 2-methylsuccinic acid is formed, which can subsequently react with hydrogen and in situ dehydratisation further to 3-methylpyrrolidone or 4-methylpyrrolidone. The hydrogenation reaction in step b2) may be performed as a two-step process, wherein in a first step the ammonium salt of itaconic acid is reacted with hydrogen to yield the ammonium salt of 2-methylsuccinic acid in situ, and in a second step the ammonium salt of 2-methylsuccinic acid is reacted with hydrogen to yield 3-methylpyrrolidone and/or 4-methylpyrrolidone. The ammonium salt of 2-methylsuccinic acid can be further converted directly or first be separated from any resulting water. The water need not be removed, and the hydrogenation reaction of step b2) may be performed as a one-step process. The hydrogenation catalysts are able to catalyse both reactions with good results.

It was however found that water resulting as a by-product from the reaction of 2-methylsuccinic acid ammonium salt with hydrogen to 3-methylpyrrolidone and/or 4-methylpyrrolidone may limit the yield. Removing the water resulting from step b2) proved advantageous and provided higher yields. Removing the water for example was found to enhance the yield by more then 75%. The water formed by dehydration as a couple product of the reaction may be removed by separation methods customarily employed for such separations such as distillation or rectification.

In embodiments of the method performed without a solvent, the hydrogenation catalyst may be selected from the group consisting of nickel, palladium, platinum, rhodium, ruthenium and combinations thereof. Preferably the hydrogenation catalyst in these embodiments is selected from the group of palladium, platinum, rhodium and ruthenium. Preferred catalysts are selected from ruthenium and rhodium. A most preferred hydrogenation catalyst is ruthenium. Ruthenium and rhodium provided best results in view of a high yield of 3- and 4-methylpyrrolidones also using a substrate melt, as in embodiments reacted in a solvent. Ruthenium and rhenium may be supported, for example on carbon or aluminum oxide. A most preferred hydrogenation catalyst is ruthenium on aluminum oxide or ruthenium on carbon, also denoted "Ru/Alox" and "Ru/C" or "Ru@Alox" and "Ru@C".

In embodiments of the method performed without a solvent, NH₃ gas may be applied with a pressure in a range from ≥ 0.5 bar to ≤ 50 bar, preferably in a range from ≥ 1 bar to ≤ 10 bar. At a pressure in such ranges a fast and complete reaction with itaconic acid could be observed.

In embodiments of the method performed without a solvent, the hydrogen may be applied with a pressure in a range from ≥ 100 bar to ≤ 300 bar, preferably in a range from ≥ 150 bar to ≤ 200 bar. Using hydrogen pressure in these ranges an advantageous high yield of 3- and 4-methylpyrrolidones could be achieved.

Both embodiments of the present invention, performed in a solvent or solvent-free in a substrate melt, can be used on an industrial scale as well as on a small scale and/or are easily adaptable to various reactors and respective demands of the course of a reaction in an industrial production process. A method can be provided which allows amination of itaconic acid in aqueous solution or solvent-free in the substrate melt using gaseous NH₃ and commercially available heterogeneous catalysts. A particularly preferred catalyst is Ru/C or Ru/Alox, preferred reaction conditions are 200 °C and 150 bar hydrogen pressure. The reaction can be carried out with particularly good yield within 4 to 16 hours.

Unless otherwise defined, the technical and scientific terms used herein have the same meaning as commonly understood by one of ordinary skill in the art to which this invention belongs.

Additional details, characteristics and advantages of the object of the invention are disclosed in the following description of the respective Examples which serve for illustrating the present invention.

### Examples

The reductive amination of itaconic acid yields a mixture of 3-methylpyrrolidinone and 4-methylpyrrolidone: 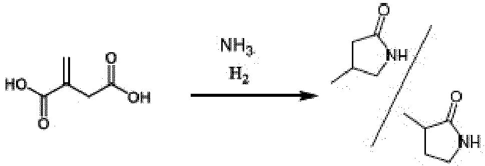
CHN analysis
CHN elemental analysis was conducted at a Vario E11 provided by the company Elementar.

### HPLC analysis

The reaction composition was analyzed using a High Performance Liquid Chromatography (HPLC) system (LC-20AD, Shimadzu) equipped with a refractive index detector as well as an organic acid resin column (Polystyrene-divinylbenzene-copolymer (PS-DVB), CS-Chromatography) maintained at 50 °C. 5 µL sample injections were made and a mobile phase of 2 mM trifluoroacetic acid in water was used with a flow rate of 1 mL min⁻¹.

### Examples 1-31

Reductive amination of itaconic acid in the molten state

### a) Synthesis of itaconic acid ammonium salt

Itaconic acid in solid phase was contacted with gaseous NH₃ to yield the ammonium salt of itaconic acid. For the gas phase amination, 10 g of itaconic acid were introduced into a batch autoclave and in solid phase 1 eq of ammonia gas, referring to itaconic acid, was applied. The temperature in the autoclave was raised to 100 °C and kept constant during the reaction. After the respective reaction time, the autoclave was cooled and vented. The reaction product was characterized by pH measurements and CHN analysis. The results are shown in the following Table 1:

**Table 1:**

| Example | reaction time [h] | pH | C [%] | H [%] | N [%] |
|---|---|---|---|---|---|
| 1 | 1 | 5.930 | 35.13 | 7.197 | 14.480 |
| 2 | 2 | 5.330 | 38.04 | 7.066 | 16.150 |
| 3 | 3 | 5.455 | 34.92 | 6.883 | 16.270 |

The results show that the ammonium salt of itaconic acid ammonium salt was received.

### b) Reductive amination of itaconic acid ammonium salt in a two-step process

### step 1: reacting itaconic acid ammonium salt with hydrogen to yield 2-methylsuccinic acid ammonium salt

The hydrogenation of itaconic acid ammonium salt was conducted in a stainless steel or 50 mL Hastelloy autoclave. 40 mg of catalyst and 1.5 g of itaconic acid ammonium salt were weighted and transferred to the autoclave. The autoclave was then pressurized with H₂ to 20 bar. Subsequently, the reaction mixture was heated to 200 °C and kept at this temperature for the reaction time. After reaction times of 1, 2 or 4 h, the autoclave was cooled and expanded. The reaction solution was analyzed by ¹H-NMR with diglyme as an internal standard and quantified.

The results for the respective catalysts and reaction times are shown in the following Table 2. "Pt/C" and "Pd/C" means platinum or palladium, in a concentration of 5 wt% referring to the support on carbon (Aldrich), and "Ru/Alox" means ruthenium catalyst (5 wt%) supported on aluminum oxide (Johnson Matthey). "Raney-Ni" refers to Raney nickel (Alfa Aesar).

**Table 2:**

| Example | catalyst | reaction time [h] | Yield [%] |
|---|---|---|---|
| 4 | Pt/C | 4 | 100 |
| 5 | Raney-Ni | 4 | 100 |
| 6 | Pd/C | 4 | 100 |
| 7 | Ru/Alox | 4 | 100 |
| 8 | Pt/C | 2 | 100 |
| 9 | Raney-Ni | 2 | 100 |
| 10 | Pd/C | 2 | 100 |
| 11 | Ru/Alox | 2 | 100 |
| 12 | Pt/C | 1 | 100 |
| 13 | Raney-Ni | 1 | 100 |
| 14 | Pd/C | 1 | 100 |
| 15 | Ru/Alox | 1 | 100 |

The hydrogenation of itaconic acid ammonium salt to 2-methylsuccinic acid ammonium salt was completed after one hour reaction time, and no difference in the reactivity of the catalysts was seen.

The ammonium salt of 2-methylsuccinic acid was separated from water by evaporation and cristallization, dried, and used as a dry solid in the following hydrogenation reaction.

### step 2: reacting 2-methylsuccinic acid ammonium salt with hydrogen to yield 3-methylpyrrolidone and 4-methylpyrrolidone

The hydrogenation of 2-methylsuccinic acid ammonium salt was conducted in stainless steel or hastelloy 50 mL autoclaves. 1.5 g of the ammonium salt of methylsuccinic acid were filled into the glass inlet of the autoclave. 2.6 wt%, referring to the amount of used substrate, of the respective catalyst were added. 2.6 wt% correspond to about 40 mg of catalyst. The glass inlet was transferred to the autoclave and the autoclave was closed. Alternatively the solution was directly weighted in the autoclave without the use of a glass inlet. Hydrogen was charged at room temperature and the autoclave subsequently was heated to 200 °C. After the reaction

times given in the following table 3, the autoclave was cooled and expanded. The obtained reaction solution was analyzed by HPLC analysis.

The results for the respective catalysts and reaction times are shown in the following Table 3. "Pt/C" (5 wt%) and "Ru/C" (5 wt%) were supplied by Aldrich, and "Rh/C" (5 wt%) and "Ru/Alox" (5 wt%) by Johnson Matthey. The yield of pyrrolidones refers to the combined amount of 3-methylpyrrolidin-2-one and 4-methylpyrrolidin-2-one.

**Table 3:**

| Example | catalyst | pressure [bar] | reaction time [h] | Yield [%] pyrrolidone |
|---|---|---|---|---|
| 16 | Pt/C | 100 | 3 | 6.6 |
| 17 | Pt/C | 125 | 3 | 6.4 |
| 18 | Pt/C | 150 | 3 | 9.9 |
| 19 | Pt/C | 150 | 10 | 19.0 |
| 20 | Pt/C | 150 | 17 | 28.6 |
| 21 | Rh/C | 150 | 3 | 14.5 |
| 22 | Rh/C | 150 | 17 | 33.1 |
| 23 | Ru/C | 150 | 3 | 16.2 |
| 24 | Ru/C | 150 | 8 | 62.5 |
| 25 | Ru/C | 150 | 10 | 50.8 |
| 26 | Ru/Alox | 150 | 3 | 37.7 |
| 27 | Ru/Alox | 150 | 8 | 58.5 |
| 28 | Ru/Alox | 150 | 10 | 60.1 |
| 29 | Ru/Alox | 150 | 17 | 42.4 |

It can be taken from the results given in table 3, that particularly rhodium and ruthenium catalysts greatly increased the reaction efficiency.

### c) reductive amination of itaconic acid ammonium salt to 3- and 4-methylpyrrolidone as a one-step reaction

The one-step reaction of itaconic acid ammonium salt to 3- and 4-methylpyrrolidone was conducted in stainless steel or hastelloy 50 mL autoclaves. 1.5 g of the ammonium salt of itaconic acid were transferred to the autoclave, and 2.6 wt% of ruthenium catalyst supported on aluminum oxide ("Ru/Alox" 5 wt%, Johnson Matthey) were added. Hydrogen was charged at room temperature and the autoclave subsequently heated to 200 °C. After the reaction, the autoclave was cooled and expanded and the obtained reaction solution was analyzed by HPLC analysis. The results for reaction times of 2 and 4 hours are shown in the following Table 4.

**Table 4:**

| Example | catalyst | pressure [bar] | reaction time [h] | Yield [%] pyrrolidone |
|---|---|---|---|---|
| 30 | Ru/Alox | 150 | 2 | 22.39 |
| 31 | Ru/Alox | 150 | 4 | 24.21 |

It can be taken from the results given in table 4, also the one-step process showed good yields of pyrrolidones.

### Comparative examples

### Reacting 2-methylsuccinic acid ammonium salt with hydrogen using Co or Cu as catalysts

The hydrogenation of 2-methylsuccinic acid ammonium salt was performed as described above in example 1b), except for the use of differing catalysts. 1.5 g of the ammonium salt of methylsuccinic acid were filled into the autoclave and 2.6 wt%, corresponding to about 40 mg, of cobalt or copper catalyst supported on carbon (5 wt%, Johnson Matthey) were added. Hydrogen was charged at room temperature and the autoclave was heated to 200 °C for 3 hours. After the reaction, the autoclave was cooled and the obtained reaction solution was analyzed by HPLC analysis. The results are shown in the following Table 5.

**Table 5: comparative example**

| Example | catalyst | pressure [bar] | reaction time [h] | Yield [%] pyrrolidone |
|---|---|---|---|---|
| A | Co/C | 150 | 3 | 0.0 |
| B | Cu/C | 150 | 3 | 0.0 |

As can be taken from the results given in table 5, cobalt and copper catalysts did not result in a yield of 3-methylpyrrolidin-2-one and 4-methylpyrrolidin-2-one.

### Examples 32-38

### Reductive amination of itaconic acid in aqueous solution

1.5 g of itaconic acid, 75 mg ruthenium catalyst supported on aluminum oxide ("Ru/Alox", 5 wt%, Johnson Matthey) and 1.5 g deionized water were transferred to a 50 ml autoclave. 75 mg ruthenium catalyst corresponded to 5 wt% referring to a total amount of 100 wt% of catalyst and substrate. Gaseous NH₃ was applied at 1 eq referring to itaconic acid at room temperature with 4.0 bar. Subsequently, hydrogen was charged until the respective pressure was reached and the autoclave was heated. After the reaction, the autoclave was cooled and the obtained reaction solution was analyzed by HPLC analysis. The results are shown in the following Table 6.

**Table 6:**

| Example | catalyst | pressure [bar] | temperature [°C] | reaction time [h] | Yield [%] pyrrolidone |
|---|---|---|---|---|---|
| 32 | Ru/Alox | 150 | 200 | 4 | 82 |
| 33 | Ru/Alox | 150 | 200 | 6 | 83 |
| 34 | Ru/Alox | 150 | 200 | 10 | > 99 |
| 35 | Ru/Alox | 150 | 200 | 16 | > 99 |
| 36 | Ru/Alox | 150 | 200 | 17 | 97 |
| 37 | Ru/Alox | 110 | 200 | 4 | 63 |
| 38 | Ru/Alox | 150 | 150 | 4 | 4 |

It can be seen from table 6 that a simple, one-pot reductive amination of itaconic acid in aqueous solution with a particular high yield of 3-methylpyrrolidin-2-one and 4-methylpyrrolidin-2-one can be achieved using ruthenium as catalyst. Even at reaction times of 4 h and a pressure of only 110 bar, very good results could be achieved.

### Example 39

### Reductive amination of itaconic acid using pyrrolidones as solvent

The reductive amination of itaconic acid was repeated as described in example 32, except that 1.5 g of a mixture of 3-methylpyrrolidin-2-one and 4-methylpyrrolidin-2-one was used as solvent. 1.5 g of itaconic acid, 75 mg Ru/Alox (5 wt%, Johnson Matthey) and 1.5 g of 3-methylpyrrolidin-2-one and 4-methylpyrrolidin-2-one were transferred to a 50 ml autoclave. Gaseous NH₃ was applied at 1 eq referring to itaconic acid at room temperature. Subsequently, hydrogen was charged to 150 bar and the autoclave was heated to 200 °C. After the reaction, the autoclave was cooled and the obtained reaction solution was analyzed by HPLC analysis. The results are shown in the following Table 7.

**Table 7:**

| Example | catalyst | pressure [bar] | temperature [°C] | reaction time [h] | Yield [%] pyrrolidone |
|---|---|---|---|---|---|
| 39 | Ru/Alox | 150 | 200 | 6 | 33 |

Example 39 shows that also other solvents such as pyrrolidones are usable as a solvent. Thus, a continuous reaction system using 3-methylpyrrolidone or 4-methylpyrrolidone as solvents is possible.

### Examples 40-42

### Reductive amination of itaconic acid in aqueous solution using rhodium, platinum or palladium catalysts

The reductive amination of itaconic acid was repeated as described in example 32, except that Rh/C (5 wt%, Alfa Aesar), Pt/C (5 wt%, Sigma Aldrich) and Pd/C (5 wt%, Sigma Aldrich) were used as catalysts. 1.5 g of itaconic acid, 75 mg catalyst and 1.5 g deionized water were transferred to a 50 ml autoclave. Gaseous NH₃ (1 eq referring to itaconic acid) was applied at room temperature. Subsequently, hydrogen was charged to 150 bar and the autoclave was heated to 200 °C. After 4 hours, the autoclave was cooled and the obtained reaction solution was analyzed by HPLC analysis. The results are shown in the following Table 8.

**Table 8: rhodium, platinum and palladium catalysts in aqueous solution**

| Example | catalyst | pressure [bar] | temperature [°C] | reaction time [h] | Yield [%] pyrrolidone |
|---|---|---|---|---|---|
| 40 | Rh/C | 150 | 200 | 4 | 32 |
| 41 | Pt/C | 150 | 200 | 4 | 13 |
| 42 | Pd/C | 150 | 200 | 4 | 9 |

Examples 40-42 show that the platinum group metals palladium, platinum, or iron metals like rhodium as well as ruthenium are useful catalysts for the reductive amination of itaconic acid in aqueous solution.

Overall, the examples show that the reductive amination of biogenic itaconic acid using gaseous NH₃ to yield 3- and 4-methylpyrrolidone by means of heterogeneous catalysis using a hydrogenation catalyst in the presence of hydrogen could be achieved with high yield in aqueous solution and solvent-free.

## Claims

1. A method for preparing methylpyrrolidones, the method comprising the steps of:
a) providing itaconic acid or a derivative of itaconic acid selected from the group of its salts, esters, imids, amids and anhydrids, and
b) reductive amination of itaconic acid or its derivative in the presence of hydrogen and a hydrogenation catalyst,
wherein itaconic acid or its derivative is reacted with NH₃ to yield 3-methylpyrrolidin-2-one and/or 4-methylpyrrolidin-2-one, and
wherein the hydrogenation catalyst is a metal catalyst selected from the group consisting of nickel, palladium, platinum, rhodium, ruthenium and combinations thereof.

2. The method according to claim 1, wherein the hydrogenation catalyst is selected from the group consisting of palladium, platinum, rhodium and ruthenium.

3. The method according to claim 1 or 2, wherein the reductive amination of itaconic acid or its derivative is performed in the presence of a solvent, preferably selected from the group consisting of water, alcohols, ethers, dimethylformamide, ammonia, ammonium hydroxide, pyrrolidones, 3-methylpyrrolidin-2-one, 4-methylpyrrolidin-2-one or mixtures thereof.

4. The method according to claim 3, wherein the hydrogenation catalyst is selected from ruthenium and rhodium.

5. The method according to claim 3 or 4, wherein the hydrogenation reaction is performed at a temperature in a range from ≥ 150 °C to ≤ 300 °C, preferably in a range from ≥ 180°C to ≤ 200°C.

6. The method according to any one of claims 3 to 5, wherein NH₃ gas is applied with a pressure in a range from ≥ 0.5 bar to ≤ 50 bar, preferably in a range from ≥ 1 bar to ≤ 10 bar.

7. The method according to any one of claims 3 to 6, wherein hydrogen is applied with a pressure in a range from ≥ 50 bar to ≤ 300 bar, preferably in a range from ≥ 150 bar to ≤ 200 bar.

8. The method according to any one of claims 3 to 7, wherein the reductive amination of itaconic acid or its derivative in a solvent is performed as a one-pot reaction.

9. The method according to claim 1 or 2, wherein the method comprises the steps of:
b1) contacting itaconic acid or its derivative in solid phase with gaseous NH₃ to yield the ammonium salt of itaconic acid; and
b2) reacting the ammonium salt of itaconic acid in the molten state with hydrogen in the presence of a hydrogenation catalyst to yield 3-methylpyrrolidin-2-one and/or 4-methylpyrrolidin-2-one.

10. The method according to claim 9, wherein the hydrogenation reaction in step b2) is performed as a two-step process, wherein in a first step the ammonium salt of itaconic acid is reacted with hydrogen to yield the ammonium salt of 2-methylsuccinic acid, and in a second step the ammonium salt of 2-methylsuccinic acid is reacted with hydrogen to yield 3-methylpyrrolidone and/or 4-methylpyrrolidone.

11. The method according to claim 9 or 10, wherein the hydrogenation reaction in step b2) is performed at a temperature in a range from ≥ 150 °C to ≤ 250 °C, preferably in a range from ≥ 150°C to ≤ 200°C.

12. The method according to any one of claims 9 to 11, wherein the hydrogenation catalyst is a metal catalyst selected from the group consisting of nickel, palladium, platinum, rhodium, ruthenium and combinations thereof, preferably selected from the platinum group metals palladium, platinum, rhodium and ruthenium.

13. The method according to any one of claims 9 to 12, wherein NH₃ gas is applied with a pressure in a range from ≥ 0.5 bar to ≤ 50 bar, preferably in a range from ≥ 1 bar to ≤ 10 bar.

14. The method according to any one of claims 9 to 13, wherein hydrogen is applied with a pressure in a range from ≥ 100 bar to ≤ 300 bar, preferably in a range from ≥ 150 bar to ≤ 200 bar.

## Patentansprüche

1. Verfahren zur Herstellung von Methylpyrrolidonen, wobei das Verfahren folgende Schritte umfasst:
a) Bereitstellen von Itaconsäure oder einem Derivat von Itaconsäure aus der Gruppe ihrer Salze, Ester, Imide, Amide und Anhydride und
b) reduktive Aminierung von Itaconsäure oder ihrem Derivat in Gegenwart von Wasserstoff und einem Hydrierkatalysator,
wobei Itaconsäure oder ihr Derivat mit NH₃ zu 3-Methylpyrrolidin-2-on und/oder 4-Methylpyrrolidin-2-on umgesetzt wird und
wobei es sich bei dem Hydrierkatalysator um einen Metallkatalysator aus der Gruppe bestehend aus Nickel, Palladium, Platin, Rhodium, Ruthenium und Kombinationen davon handelt.

2. Verfahren nach Anspruch 1, wobei der Hydrierkatalysator aus der Gruppe bestehend aus Palladium, Platin, Rhodium und Ruthenium ausgewählt wird.

3. Verfahren nach Anspruch 1 oder 2, wobei die reduktive Aminierung von Itaconsäure oder ihrem Derivat in Gegenwart eines Lösungsmittels, vorzugsweise aus der Gruppe bestehend aus Wasser, Alkoholen, Ethern, Dimethylformamid, Ammoniak, Ammoniumhydroxid, Pyrrolidonen, 3-Methylpyrrolidin-2-on, 4-Methylpyrrolidin-2-on oder Mischungen davon, durchgeführt wird.

4. Verfahren nach Anspruch 3, wobei der Hydrierkatalysator aus Ruthenium und Rhodium ausgewählt wird.

5. Verfahren nach Anspruch 3 oder 4, wobei die Hydrierungsreaktion bei einer Temperatur in einem Bereich von ≥ 150 °C bis ≤ 300 °C, vorzugsweise in einem Bereich von ≥ 180 °C bis ≤ 200 °C, durchgeführt wird.

6. Verfahren nach einem der Ansprüche 3 bis 5, wobei NH₃-Gas mit einem Druck in einem Bereich von ≥ 0,5 bar bis ≤ 50 bar, vorzugsweise in einem Bereich von ≥ 1 bar bis ≤ 10 bar, aufgepresst wird.

7. Verfahren nach einem der Ansprüche 3 bis 6, wobei Wasserstoff mit einem Druck in einem Bereich von ≥ 50 bar bis ≤ 300 bar, vorzugsweise in einem Bereich von ≥ 150 bar bis ≤ 200 bar, aufgepresst wird.

8. Verfahren nach einem der Ansprüche 3 bis 7, wobei die reduktive Aminierung von Itaconsäure oder ihrem Derivat in einem Lösungsmittel als Eintopfreaktion durchgeführt wird.

9. Verfahren nach Anspruch 1 oder 2, wobei das Verfahren folgende Schritte umfasst:
b1) Inberührungbringen von Itaconsäure oder ihrem Derivat in fester Phase mit gasförmigem NH₃ zum Erhalt des Ammoniumsalzes von Itaconsäure und
b2) Umsetzen des Ammoniumsalzes von Itaconsäure in schmelzflüssigem Zustand mit Wasserstoff in Gegenwart eines Hydrierkatalysators zum Erhalt von 3-Methylpyrrolidin-2-on und/oder 4-Methylpyrrolidin-2-on.

10. Verfahren nach Anspruch 9, wobei die Hydrierungsreaktion in Schritt b2) als zweischrittiges Verfahren durchgeführt wird, wobei in einem ersten Schritt das Ammoniumsalz von Itaconsäure mit Wasserstoff zu dem Ammoniumsalz von 2-Methylbernsteinsäure und in einem zweiten Schritt das Ammoniumsalz von 2-Methylbernsteinsäure mit Wasserstoff zu 3-Methylpyrrolidon und/oder 4-Methylpyrrolidon umgesetzt wird.

11. Verfahren nach Anspruch 9 oder 10, wobei die Hydrierungsreaktion in Schritt b2) bei einer Temperatur in einem Bereich von ≥ 150 °C bis ≤ 250 °C, vorzugsweise in einem Bereich von ≥ 150 °C bis ≤ 200 °C, durchgeführt wird.

12. Verfahren nach einem der Ansprüche 9 bis 11, wobei es sich bei dem Hydrierkatalysator um einen Metallkatalysator aus der Gruppe bestehend aus Nickel, Palladium, Platin, Rhodium, Ruthenium und Kombinationen davon, vorzugsweise aus dem Platingruppenmetallen Palladium, Platin, Rhodium und Ruthenium, handelt.

13. Verfahren nach einem der Ansprüche 9 bis 12, wobei NH₃-Gas mit einem Druck in einem Bereich von ≥ 0,5 bar bis ≤ 50 bar, vorzugsweise in einem Bereich von ≥ 1 bar bis ≤ 10 bar, aufgepresst wird.

14. Verfahren nach einem der Ansprüche 9 bis 13, wobei Wasserstoff mit einem Druck in einem Bereich von ≥ 100 bar bis ≤ 300 bar, vorzugsweise in einem Bereich von ≥ 150 bar bis ≤ 200 bar, aufgepresst wird.

## Revendications

1. Procédé de préparation de méthylpyrrolidones, le procédé comprenant les étapes qui consistent à :
a) fournir de l'acide itaconique ou un dérivé d'acide itaconique sélectionné dans le groupe constitué de ses sels, esters, imides, amides et anhydrides, et
b) réaliser une amination réductrice de l'acide itaconique ou de son dérivé en présence d'hydrogène et d'un catalyseur d'hydrogénation, dans lequel on fait réagir l'acide itaconique ou son dérivé avec du NH₃ pour produire de la 3-méthylpyrrolidin-2-one et/ou de la 4-méthylpyrrolidin-2-one, et
dans lequel le catalyseur d'hydrogénation est un catalyseur métallique sélectionné dans le groupe constitué du nickel, du palladium, du platine, du rhodium, du ruthénium et de combinaisons de ceux-ci.

2. Procédé selon la revendication 1, dans lequel le catalyseur d'hydrogénation est sélectionné dans le groupe constitué du palladium, du platine, du rhodium et du ruthénium.

3. Procédé selon la revendication 1 ou 2, dans lequel l'amination réductrice de l'acide itaconique ou de son dérivé est réalisée en présence d'un solvant, préférablement sélectionné dans le groupe constitué de l'eau, d'alcools, d'éthers, du diméthylformamide, de l'ammoniac, de l'hydroxyde d'ammonium, de pyrrolidones, de la 3-méthylpyrrolidin-2-one, de la 4-méthylpyrrolidin-2-one ou de mélanges de ceux-ci.

4. Procédé selon la revendication 3, dans lequel le catalyseur d'hydrogénation est sélectionné parmi le ruthénium et le rhodium.

5. Procédé selon la revendication 3 ou 4, dans lequel la réaction d'hydrogénation est réalisée à une température de > 150 °C à < 300 °C, préférablement de > 180 °C à < 200 °C.

6. Procédé selon l'une quelconque des revendications 3 à 5, dans lequel le gaz NH₃ est appliqué à une pression de ≥ 0,5 bar à ≤ 50 bars, préférablement de ≥ 1 bar à ≤ 10 bars.

7. Procédé selon l'une quelconque des revendications 3 à 6, dans lequel l'hydrogène est appliqué à une pression de ≥ 50 bars à ≤ 300 bars, préférablement de ≥ 150 bars à ≤ 200 bars,

8. Procédé selon l'une quelconque des revendications 3 à 7, dans lequel l'amination réductrice de l'acide itaconique ou de son dérivé dans un solvant est réalisée sous la forme d'une réaction monotope.

9. Procédé selon la revendication 1 ou 2, le procédé comprenant les étapes qui consistent à :
b1) mettre en contact l'acide itaconique ou son dérivé en phase solide avec du NH₃ gazeux pour produire le sel d'ammonium de l'acide itaconique ; et
b2) faire réagir le sel d'ammonium de l'acide itaconique à l'état fondu avec de l'hydrogène en présence d'un catalyseur d'hydrogénation pour produire de la 3-méthylpyrrolidin-2-one et/ou de la 4-méthylpyrrolidin-2-one.

10. Procédé selon la revendication 9, dans lequel la réaction d'hydrogénation à l'étape b2) est réalisée comme un processus en deux étapes, dans lequel, dans une première étape, on fait réagir le sel d'ammonium de l'acide itaconique avec de l'hydrogène pour produire le sel d'ammonium de l'acide 2-méthylsuccinique, et dans une deuxième étape, on fait réagir le sel d'ammonium de l'acide 2-méthylsuccinique avec de l'hydrogène pour produire de la 3-méthylpyrrolidone et/ou de la 4-méthylpyrrolidone.

11. Procédé selon la revendication 9 ou 10, dans lequel la réaction d'hydrogénation à l'étape b2) est réalisée à une température de ≥ 150 °C à ≤ 250 °C, préférablement de ≥ 150°C à ≤ 200°C.

12. Procédé selon l'une quelconque des revendications 9 à 11, dans lequel le catalyseur d'hydrogénation est un catalyseur métallique sélectionné dans le groupe constitué du nickel, du palladium, du platine, du rhodium, du ruthénium et de combinaisons de ceux-ci, préférablement sélectionné dans le groupe de métaux du groupe du platine comprenant le palladium, le platine, le rhodium et le ruthénium.

13. Procédé selon l'une quelconque des revendications 9 à 12, dans lequel le gaz NH₃ est appliqué à une pression de ≥ 0,5 bar à ≤ 50 bars, préférablement de ≥ 1 bar à ≤ 10 bars.

14. Procédé selon l'une quelconque des revendications 9 à 13, dans lequel l'hydrogène est appliqué à une pression de ≥ 100 bars à ≤ 300 bars, préférablement de ≥ 150 bars à ≤ 200 bars.
